# EUROPÄISCHE PATENTSCHRIFT

(11) **EP 0 338 383 B1**
(45) Veröffentlichungstag und Bekanntmachung des Hinweises auf die Patenterteilung: **24.03.1993**
(21) Anmeldenummer: 89106384.4
(22) Anmeldetag: 11.04.1989
(51) Int. Cl.: A61K 9/54, A61K 9/24

(54) **Oral zu verabreichende pharmazeutische Zubereitung mit kontrollierter Wirkstofffreisetzung und Verfahren zu deren Herstellung**
Pharmaceutical preparation to be administered orally with controlled release of active substance and method for its manufacture
Préparation pharmaceutique pour administration orale à libération contrôlée du principe actif et son procédé de fabrication

(30) Priorität: 16.04.1988 DE 3812799
(43) Veröffentlichungstag der Anmeldung: 25.10.1989
(73) Patentinhaber: SCHWARZ PHARMA AG, D-40789 Monheim (DE)
(72) Erfinder: Klokkers-Bethke, Karin, Dr.rer.nat., D-4019 Monheim/Rhld (DE); Fischer, Wilfried, Dr.rer.nat., D-5093 Burscheid (DE)

(56) Entgegenhaltungen:
- EP-A- 0 032 562
- EP-A- 0 040 590
- EP-A- 0 092 060

## Beschreibung

Die vorliegende Erfindung betrifft eine oral zu verabreichende pharmazeutische Zubereitung mit kontrollierter WirkstofffreiSetzung und Verfahren zu deren Herstellung wie sie in den Patentansprüchen einzeln gekennzeichnet ist.

Es ist bekannt, Retardarzneiformen einzusetzen, um die Einnahmefrequenz zu senken und durch Vermeidung von Plasmaspiegelspitzen Nebenwirkungen zu minimieren. Dadurch soll die Patientencompliance erhöht werden. Idealerweise soll die einmal tägliche Gabe der entsprechenden Arzneimittel angestrebt werden.
Das Erreichen des Zieles ist abhängig von:
einmal den Eigenschaften der Pharmaka selbst wie biologische Halbwertszeit, Absorptionscharakteristik und Toleranzentwicklung;
als auch den Eigenschaften der verwendeten Retardarzneiform wie Wirkstofffreisetzungsprofil und Aufenthaltsdauer in den resorbierenden Magen-Darmabschnitten.

Standardretardformen setzen den Wirkstoff in weniger als 8 Stunden frei, "langretardierte" Arzneiformen besitzen eine Freisetzungszeit von mehr als 8 Stunden. Diesen herkömmlichen Retardformen ist die zeitlich annähernd konstante Freisetzungsgeschwindigkeit gemeinsam.

Pharmaka, die bei chronischer Anwendung Toleranzbildung erwarten lassen (z.B. Nitrate, Corticosteroide) bzw. deren Wirkung oder unerwünschte Wirkung von der tageszeitlich unterschiedlichen Applikation abhängig ist (z.B. Glucocorticoide), sollten in Intervallen mit wirkstoffhaltigen und quasi "wirkstofffreien" Blutspiegeln, zumindest solchen unterhalb der Toleranz- bzw. Toxizitätsschwelle angewendet werden (J. Urquhart, Delivery Systems & pharmacodynamics in new drug research & development, **BIOTECH 1985 USA,** Online Publications, Pinner, UK 1985, p. 321-331). Zur einmal oder zweimal täglichen Gabe in langretardierten Formulierungen, die den Wirkstoff gleichmäßig abgeben, sind sie nicht geeignet. Hierbei besteht die Gefahr, daß sich bei konstanten Plasmaspiegeln sehr schnell Wirkungsabschwächungen oder Nebenwirkungen durch physiologische Gegenregulationen einstellen. Arzneiformen, die nach einmaliger Verabreichung mehrere Plasmaspiegelspitzen und -täler erzeugen, würden z.B. die Toleranzentstehung vermindern oder sogar verhindern. Darüberhinaus würde die Einnahmehäufigkeit gesenkt und die Compliance erhöht.
Mit herkömmlichen, über die Zeit mit konstanter Geschwindigkeit freisetzenden Retardformen können derartige pulsierende Plasmaspiegel nicht erreicht werden: Bei richtiger Ausführung der dem derzeitigen Stand der Technik entsprechenden Retardformen wird ein mehr oder weniger konstantes Plasmaspiegelplateau erzeugt, das durch Zugabe einer Initialdosis in Fällen, wo dies erforderlich ist, schnell erreicht wird. Nach einer bestimmten Zeit, die durch die Transitzeit der Retardform durch resorbierende Magen-Darm-Abschnitte in Kombination mit der gewählten Freisetzungszeit bestimmt wird, fallen die Plasmaspiegel bis zum nächsten Einnahmezeitpunkt ab. Ein erneutes Anfluten der Wirksubstanz bei einmaliger Einnahme ist dabei nicht möglich (D. Ganderton, Drug Design and Delivery, **2,** 1-7 (1987). Gerade dies wäre aber zur Erzeugung fluktuierender Plasmaspiegel notwendig.

Pharmaka, die keiner Wirkungsabschwächung bei konstanten Plasmaspiegeln unterliegen, können in der Regel durch Verabreichung in einer langretardierten, d.h. kontinuierlich freisetzenden Retardarzneiform keine gleichmäßigen Plasmaspiegelverlaüfe über 24 Stunden erreichen. Im Mittel 6-8 Stunden nach der Applikation gelangen Retardarzneiformen, zumindest nach Nüchterngabe, unabhängig davon, ob sie als single oder multiple dose Formen eingesetzt werden, in untere Dünndarmabschnitte wie das distale Illeum oder ins Colon. Für die überwiegende Anzahl von Pharmaka ist in diesen Abschnitten die Resorptionsgeschwindigkeit gegenüber dem Dünndarmbereich reduziert. Setzt eine Retardform den Wirkstoff mit gleichbleibender Geschwindigkeit frei, führt das unvermeidlich zu einem Abfall der Plasmaspiegel. Außer der pharmakonspezifischen Absorptionscharakteristik führen im unteren Dünndarm und im Colon die Reduzierung des Wassergehaltes, geänderte Lösungs- und Diffusionsverhältnisse und die zunehmende Viskosität des Darminhaltes zu einer allgemeinen unspezifischen Resorptionsverschlechterung von Wirkstoffen aus verschiedenen Darreichungsformen. Es sind nur wenige Beispiele bekannt wie z.B. Theophyllin, Metoprolol (A.L. Golub et al. J.Allergy.Clin.Immunol. **78,** 689-694 (1986); G. Ragnarson et al. Development of a new controlled release Metoprolol Product, 6th Pharmaceutical Technology Conference Canterbury, England, 1987, Vol I, p. 65-81), bei denen gleichmäßige Plasmaspiegel über 24 Stunden mit langretardierten Arzneiformen erreicht wurden.

Langretardierte Arzneiformen führen zu einer Wirkstofffreisetzung über eine solche Zeitspanne, daß ein Teil der Dosis in weniger gut absorbierende Darmabschnitte gelangt. Diese Teildosis ist nicht in dem Maße für den Organismus nutzbar wie die in den übrigen Darmabschnitten freigesetzte. Bioverfügbarkeitsverluste sind die Folge. Es wird im Verhältnis weniger resorbiert als aus höhergelegenen Darmabschnitten. Aus diesem Grunde wird empfohlen (R. Khosla und S.S. Davis, J.Pharm.Pharmacol, **39,** 47-49(1987); D.Ganderton, Drug Design and Delivery, **2,** 1-7(1987)), eine Retardierungszeit von 8-12 Stunden nicht zu überschreiten, um nicht einen zu hohen Bioverfügbarkeitsverlust in Kauf nehmen zu müssen. Der kann dann auftreten, wenn die Freisetzungszeit in vivo so lang wird, daß die Retardform mit einem noch nicht resorbierten Dosisanteil bereits wieder ausgeschieden wird.
Vorteilhaft wären Arzneiformen, die unter Berücksichtigung der entsprechenden Resorptionsgeschwindigkeiten Arzneistoffe dem Organismus so anbieten, daß bei einmaliger Gabe pro Tag über einen gewünschten Zeitraum gleichmäßige Plasmaspiegel resultieren. Dies kann bedeuten, daß die Arzneiformulierung z.B. im Colon ihren Arzneistoff mit höherer Geschwindigkeit freisetzen muß als in den übrigen Darmabschnitten.

Arzneiformen, die die Gesamt- oder eine Teildosis des Wirkstoffes im Dickdarmbereich freisetzen sollen, müssen so konzipiert sein, daß sie unter Zuhilfenahme einer wirkstoffabgabefreien Zeitspanne (Lagphase) oder durch ein vom Dickdarmmilieu abgeleitetes Signal mit der Wirkstofffreigabe beginnen. Im ersten Fall muß das freisetzungsfreie Intervall so bemessen sein, daß nach der zu erwartenden Transitzeit von der Einnahme bis zum Erreichen des Colons hier der Wirkstoff zur Verfügung steht.
Mögliche vom Dickdarm abgeleitete Signale können colonspezifische Enzyme sein. Beispielsweise ist bekannt, daß bevorzugt im Colonbereich azogruppenhaltige Substanzen bakteriell reduziert werden (H. Schröder und D.E.S. Campbell, Clin.Pharmacol.Ther., **13,** 539 (1972)).
In Abhängigkeit vom verwendeten Pharmakon kann seine Resorptionsgeschwindigkeit aus dem Colon selbst so gering sein, daß eine retardierte Wirkstoffabgabe im Colonbereich selbst nicht sinnvoll ist. Arzneiformen, die langsam aus den unteren Darmabschnitten resorbiert werden, müssen hier schnell freigesetzt werden. Durch die dadurch erreichte Erhöhung der Wirkstoffkonzentration im betreffenden Resorptionsgebiet kann bei anzunehmender passiver Diffusion, dem Fick'schen Gesetz entsprechend, der Wirkstofftransport durch die Dickdarmschleimhaut erhöht werden.
In der Literatur sind Versuche bekannt, das Colon als Zielorgan für die Wirkstoffabgabe mit Retardarzneiformen zu erreichen. So beschreiben die DOS 3538038 A1, DOS 3538040 A1 und die US-PS 4,627,851 osmotische Systeme, die geeignet sein sollen, den in ihnen enthaltenen Wirkstoff erst im Colon freizusetzen. Die Freisetzungsgeschwindigkeit ist jedoch konstant und außerdem so gering (zwischen 10 und etwa 80% in 26 bis 28 Std), daß die Gefahr der Ausscheidung der noch pharmakonhaltigen Formulierung besteht. In der Praxis resultiert der mögliche Verlust eines unvorhersehbaren Dosisanteils und damit eine unkontrollierbare Bioverfügbarkeitsminderung.
Dieses nicht zu beeinflussende Verhalten der so konzipierten osmotischen Systeme widerspricht dem Grundgedanken einer Arzneiform mit *kontrollierter* Wirkstoffabgabe: Die Transitzeit solcher single dose Arzneiformen zum Colon ist im Einzelfall dann unvorhersehbar, wenn die Applikation nach oder zusammen mit einer Mahlzeit erfolgt (S.S. Davis et al. Int. J. Pharmaceutics, **21,** 331-340 (1984)). Sie kann im Extremfall bis über 10 Stunden betragen und ist sehr starken interindividuellen Variationen ausgesetzt (H. Bechgaard und K. Ladefoged, J.Pharm.Pharmacol., **33,** 791-792 (1981)). Die Zeit, nach der die Wirkstoffdosis zur Resorption zur Verfügung steht, ist somit also unkontrollierbar. Dies kann zu einem nicht akzeptablen Wirksamkeitsverlust führen. Damit ist die Arzneimittelsicherheit nicht gewährleistet.
Osmotische Systeme, wie die der o.g. Patentschriften sind dafür bekannt, daß sie im Einzelfall starke schleimhautschädigende Effekte haben (Schneidbrennereffekt) (Pharm. Ztg. **128,** 1845 (1983)), die zu Durchbrüchen durch die Magen- bzw. Dünndarmwand führen können. Diese sind durch die sehr kleine Austrittsöffnung für das Pharmakon und damit durch hohe Wirkstoffkonzentrationen an der Austrittsstelle bedingt.
Darüberhinaus ist die Herstellung osmotischer Systeme aufwendig und teuer. Die Herstellung macht den Einsatz komplizierter Technologien erforderlich (z.B. Lasertechnik), um mit der gebotenen Präzision Löcher in den osmotisch wirksamen Membranen zu erzeugen.

C. Bogentoft et al., Acta Pharm. Suec. **20,** 311-314 (1983) und EPA 0040 590 beschreiben eine Arzneiform auf der Basis von multiple dose Wirkstoffträgern, die Wirkstoffe für die lokale Behandlung von Darmentzündungen (Salicylazo-sulfapyridin, 5-Aminosalicylsäure, Cholestyramin) im Colon freisetzen sollen. Entsprechend der Patentanmeldung werden Wirkstoffkerne mit einer Schicht, enthaltend ein anionisches carboxylhaltiges Polyacrylat und ein quartäre Ammoniumgruppen enthaltendes Polyacrylat, überzogen. Die Wirkstoffabgabe aus diesem System ist stark pH-abhängig. Dabei dient die wasserunlösliche äußere Membran dazu, die Wasserdiffusion in die Granula hinein und die Wirkstoffdiffusion aus den Granula heraus zu verzögern. Dies zeigt auch die Dicke dieser Schicht, die 15-90% des gesamten Überzuges ausmachen kann. Die Arzneiform stellt eindeutig ein diffusionskontrolliertes System dar (äußere wasserunlösliche Schicht), bei dem die Membrandicke sich pH-abhängig vermindert. Im sauren Medium ist eine maximal dicke Diffusionsschicht (innere magensaftunlösliche und äußere Schicht), im neutralen oder alkalischen Medium nach Auflösung der inneren Schicht nur die verbleibende äußere Schicht diffusionskontrollierend. Entsprechend der Gesamtschichtdicke ist die sich einstellende Lagphase pH-abhängig.
Diese Form wird nicht den interindividuellen pH-Variationen des Gastrointestinaltraktes gerecht. Die o.g. Forderung nach einer im Bedarfsfalle schnellen Wirkstoffabgabe an das Colon wird nicht erfüllt.

EP 0 032 562 beschreibt Dipyridamol-Retardformen, die den Wirkstoff im Magen-Darm-Trakt in geregelter Weise retardiert abgeben. Dies wird dadurch erreicht, daß Dipyridamol und Säure gemischt werden und anschließend verkapselt werden. Auf diese Weise soll die Unlöslichkeit von Dipyridamol bei höherem pH in tieferen Darmabschnitten durch Zusatz von sauer wirkenden Stoffen ausgeglichen werden, eine rasche Neutralisation der Säure durch den im großen Überschuß vorhandenen Darmsaft vermindert und die sich viel schneller auflösende Säure ausreichend lange zurückgehalten werden. Es handelt sich hierbei um eine wirkstoffangepaßte Diffusionshülle, die durch alleinige pH-Steuerung im Verdauungstrakt den Wirkstoff freigeben soll. Die alleinige pH-Steuerung ist jedoch aufgrund großer inter- und intraindividueller Schwankungen und vor allem fehlender pH-Sprünge im Gastrointestinaltrakt ein ungeeignetes Freisetzungssignal.
EP-A 0 092 060 beschreibt konventionelle Retardpellets, die aus einem Kern und aufeinander aufgebrachten Lackschichten aus unlöslichen Polymeren bestehen.
Die Freisetzung des Wirkstoffes erfolgt durch Diffusion; die Dauer der Freisetzung ist nur von der Schichtdichte abhängig und dann ab Erreichen eines pH 5,5 gleichförmig. Dabei wird dieser pH-Wert als gleichbedeutend mit Magensaftresistenz angesehen, so daß es sich hierbei um eine Arzneiform handelt, aus der der Wirkstoff direkt nach Verlassen des Magens durch Diffusion freigesetzt wird. Eine gezielte Steuerung des Beginns der Wirkstofffreisetzung ist mit dem in der EP-A 0 092 060 beschriebenen Pelletaufbau nicht zu erreichen.

M. Saffran et al., Science, **233,** 1081-1084 (1986) beschreibt ein Überzugsmaterial für Tabletten, das durch reduzierende Colonbakterien gespalten wird. Das vorher unlösliche Polymer löst sich im Colon auf und gibt die Tablettenkerne frei. Die Autoren schlagen dieses System für die orale Insulintherapie vor. Die Freisetzungsgeschwindigkeit aus dieser Form ist unmittelbar von der Bakterienbesiedlung des Colons abhängig und wird damit starken inter- und intraindividuellen Variationen unterliegen, die gerade die Therapiesicherheit bei Insulin gefährden.
EP 227814-A beansprucht, die Freisetzungsrate an dem Punkt zu erhöhen, wo die Resorptionsfähigkeit des Organismus reduziert wird. Die Arzneiform ist eine Matrixtablette. Die Matrix setzt in vitro bei Verwendung von bimodal verteilter Hydroxypropylmethylcellulose bimodal frei. Eine in vivo Korrelation fehlt, wobei gerade bei Hydrogelbildnern die Quellung und damit die Wirkstoffabgabe stark vom umgebenden Medium , seiner Ionenstärke, Ionenart und Nahrung, Art der Nahrung sowie mechanischen Belastungen abhängig ist. Die monolithische Arzneiform der Matrixtablette ist hinsichtlich der Transitzeit im Gastrointestinaltrakt weniger gut vorhersehbar als multiple dose Formen. Ob die Erhöhung der Freisetzungsrate in vitro auch in vivo zur richtigen Zeit am richtigen Ort erfolgt, ist nicht zuverlässig voraussagbar. Wie aus den Ausführungsbeispielen hervorgeht, sind wirkstoffabgabefreie Zeitintervalle nicht zu erzeugen.
EP 202051-A beansprucht, über die Kombination verschieden freisetzender Pelletsorten einer Kapselfüllung ebenfalls eine 1x-tägliche Gabe zu erreichen. Es handelt sich hierbei um normale Retardpellets, die ebenfalls keine wirkstoffabgabefreien Intervalle gewährleisten.

FR 2565107-A beschreibt aus konzentrischen Schichten von Wirkund Hilfsstoffen aufgebaute Granula mit einer angeblichen Arzneistofffreigabe am gewünschten Wirkort, wobei der Beleg hierfür fehlt und bei diesem Pelletaufbau, der kein spezifisches wirkstoffabgabekontrollierendes Element enthält, auch der Grund für ein solches Verhalten nicht erklärbar ist. Phasen ohne Wirkstofffreisetzung sind mit dieser Form nicht erzielbar.
DE 3424648-A und DE 3329265-A beschreiben normale, kontinuierlich freisetzende Matrixarzneiformen, die die Eignung für die 1x-tägliche Gabe beanspruchen, ohne unterschiedliche Resorptionsverhältnisse an verschiedenen Orten im Gastrointestinaltrakt zu berücksichtigen. Wie aus den Ausführungsbeispielen hervorgeht, sind wirkstoffabgabefreie Zeitintervalle nicht zu erzeugen
DE 3331262 und EP 0225189 beanspruchen, Arzneistoffe an definierten Orten im Verdauungstrakt durch alleinige pH-Steuerung freizusetzen. Die alleinige pH-Steuerung ist aufgrund großer inter- und intraindividueller Schwankungen und vor allem fehlender pH-Sprünge im .Gastrointestinaltrakt ein ungeeignetes Freisetzungssignal.

Aufgabe der vorliegenden Erfindung ist es, eine Arzneiform zu schaffen , die die vorstehend beschriebenen Gefahren und Nachteile beseitigt und weitgehend unabhängig von u.a. interindividuellen Variationen des pH-Wertes und der Bakterienflora im Gastrointestinaltrakt den inkorporierten Wirkstoff an einem vorbestimmten Ort oder zu einer vorbestimmten Zeit freisetzt und eine optimale Bioverfügbarkeit sicherstellt. Die Arzneiform soll nach einer im voraus festzulegenden Zeit mit der Wirkstoffabgabe beginnen, um bei Bedarf in Kombination mit sofort freisetzenden Dosisanteilen fluktuierende Plasmaspiegel für eine bedarfsangepaßte Therapie zu erzeugen.

Die Lagphase vor der Freisetzung einer definierten Dosis soll insbesondere so bemessen sein, daß der obere Colonbereich (colon ascendens) sicher erreicht und der Wirkstoff hier mit einer vorbestimmten Rate an das Colon abgegeben wird. Die Abgaberate soll so bemessen sein, daß dem jeweiligen Therapiebedarf angemessen hohe und langanhaltende Plasmaspiegel, bzw. Konzentrationen im Darmlumen bei lokal wirksamen Stoffen erzielt werden. In Kombination mit einem Dosisanteil, entsprechend der herkömmlichen Retardierung, wird damit die tägliche Einmalgabe von geeigneten Arzneistoffen ermöglicht.

Diese Aufgabe wird erfindungsgemäß dadurch gelöst, daß einen Kern, eine wirkstoffhaltige Schicht, enthaltend mindestens eine pharmakologisch aktive Substanz, eine Membran, eine Säureschicht, bestehend aus mindestens einer physiologisch unbedenklichen Säure und eine Verbundmembran bestehend aus zwei diskreten Schichten, deren eine von einem pH-sensitiven Polymer und deren andere von einem unlöslichen Polymer gebildet wird, umhüllen.
Nach einer Ausbildung besteht der Kern selbst aus mindestens einer pharmakologisch aktiven Substanz.
Eine weitere Ausbildung ist, daß die wirkstoffhaltige Schicht mindestens eine beliebige pharmakologisch aktive Substanz enthält, die Membran pH-unabhängig oder pH-abhängig löslich ist, die Säureschicht aus Natriumdihydrogenphosphat, Citronensäure, Weinsäure, Bernsteinsäure, Fumarsäure, ohne darauf beschränkt zu sein, und/oder Mischungen davon und die Verbundmembran aus einem pH-sensitiven Polymer und einem unlöslichen Polymer besteht. Nach einer anderen Weiterbildung der Erfindung ist die Membran Celluloseacetatphthalat.
Vorteilhaft ist, daß das pH-sensitive Polymer aus Copolymeren aus Methacrylsäure und Methacrylsäuremethylester, Carboxymethylethylcellulose, Celluloseacetatphtalat, Celluloseacetylphtalat, Cellulosediacethylphtalat, Cellulosetriacethylphtalat, Hydroxypropylmethylcellulosephtalat, Natriumcelluloseacetatphtalat, Celluloseesterphtalat, Celluloseetherphtalat, Celluloseesteretherphtalat, Hydroxypropylcellulosephtalat, Methylcellulosephtalat, Keratin, Keratin-Sandarac-Tolubalsam, Salol, Salol-ß-naphtylbenzoat und Acetotannin oder Salol mit Perubalsam, ohne darauf beschränkt zu sein, oder Mischungen dieser Polymere untereinander und/oder löslichen oder unlöslichen Polymeren besteht. Weiterhin vorteilhaft ist, daß das unlösliche Polymer Celluloseether, Copolymerisat aus Acryl- und Methacrylsäureestern mit quartären Ammoniumgruppen oder Polyvinylacetat, ohne darauf beschränkt zu sein, und/oder Mischungen davon und/oder Anteile von löslichen Polymeren enthaltend, ist.
Besonders vorteilhaft ist, daß die Verbundmembran porenbildende Substanzen enthält.
Weiterhin ist vorteilhaft, daß der Polymeranteil der unlöslichen Membran der Verbundschicht 1 - 30 Gew.-% beträgt.
Nach einer besonders vorteilhaften Weiterbildung der Erfindung enthält die wirkstoffhaltige Schicht zusätzlich zu mindestens einer pharmakologisch aktiven Substanz mindestens einen Absorptionsförderer und/oder mindestens einen Solubilisator.
Weiterhin können die wirkstoffhaltige und die Säureschicht zu einer Schicht vereint sein, daß diese eine mindestens eine pharmakologisch aktive Substanz und mindestens eine physiologisch unbedenkliche Säure enthaltende Wirkstoff-Säuremischung ist.
Nach einer anderen Ausbildung der Erfindung ist die Membran abwesend.
Weiterhin ist die Arzneiform eine Tablette, ein Pellet, eine Kapsel, ein Granulum, eine Pille oder ein Dragee.
Ferner kann die erfindungsgemäße pharmazeutische Zubereitung hergestellt werden, indem auf einen physiologisch unbedenklichen Kern mindestens eine pharmakologisch aktive Substanz aufgebracht wird, auf die gegebenenfalls eine Membranschicht und hierauf eine Säureschicht aufgetragen wird, auf die die Verbundmembran aufgebracht wird.
Nach einer vorteilhaften Verfahrensausgestaltung werden die verschiedenen Schichten durch Agglomerieren, Aufsprühen in der Wirbelschicht, Auftragen per Hand, durch Pulverdosierer, durch Aufpressen, Verpressen, Aufkleben, Extrudieren, Tauchen und/oder Aufwalzen auf den wirkstoffhaltigen Kern aufgebracht. Schließlich eignet sich die erfindungsgemäße pharmazeutische Zubereitung zur Therapie von Krankheiten.

Im Folgenden wird die Erfindung näher beschrieben.
In vielen Untersuchungen wurde gefunden, daß die Passagezeit eines Pharmakons durch den Dünndarm mit etwa 3 - 4 Stunden nahezu konstant ist; (S.S. Davis, J. Contr. Release, **2,** 27-38 (1985) und J.G. Davis et al., Gut, **27,** 886-892 (1986)). Diesen Zeitraum galt es für ein abgabefreies Intervall zu nutzen. Das Verlassen des Magens und der damit verbundene pH-Wechsel sollte als Signal für den Beginn der genannten Zeitspanne dienen. Durch Integrieren eines internen Puffersystems in eine mit einer Verbundmembran überzogene Arzneiform wird die Ablösung der pH-sensitiven Membran zeitlich verzögert und so eine Lagphase erzeugt. Als Prüfmedium diente eine Flüssigkeit, deren pH-Wert oberhalb des zur Auflösung des pH-sensitiven Anteils der Verbundmembran benötigten lag.
Überraschenderweise ermöglicht nur eine Verbundschicht in erfindungsgemäßer Zusammensetzung im Zusammenwirken mit einer in der Arzneiform enthaltenen physiologisch unbedenklichen Säure die Zeitsteuerung. Weiterhin überraschend ist die Tatsache, daß der unlösliche Anteil der Verbundmembran in einer solch geringen Schichtdicke aufgetragen werden kann, daß sie nicht als freigabekontrollierende Membran für den Wirkstoff wirkt.

Als Arzneiformen kommen feste Darreichungsformen wie Tabletten, Pellets, Kapseln, Granula, Pillen und Dragees in Betracht. In hervorragender Weise sind Pellets und Granula als Wirkstoffträger geeignet. Diese Formen unterliegen einer reproduzierbaren Bewegungskinetik im Magen-Darm-Kanal. Die Transitzeit vom Verlassen des Magens bis zum Erreichen des Colons ist gut vorhersehbar und unabhängig von der Nahrungsaufnahme (S.S. Davis et al., Int. J. Pharmaceutics, **21,** 331-340 (1984)). Sie beträgt typisch 3-4 Std (J.G. Hardy, J.Nucl.Med., **25,** 59 (1984)).
Arzneiformen der erfindungsgemäßen Zusammensetzung zeichnen sich dadurch aus, daß nach dem Verlassen des Magens beliebig lange Zeitintervalle mit geringster Wirkstofffreisetzung hergestellt werden können. Nach dieser Periode wird der Wirkstoff mit beliebig einstellbarer Geschwindigkeit freigesetzt.

Der Aufbau der Arzneiform richtet sich dabei nach den Anfordernissen an die Freisetzung nach Erreichen der Zielorte. So kann auf einen wirkstoffhaltigen Kern (in der Abbildung mit I/II gekennzeichnet) eine pH-unabhängig oder pH-abhängig auflösende Membran (Wirkstoffabgabesteuerung)(III) aufgebracht werden. Auf diese Membran wird die physiologisch unbedenkliche Säure(IV) und hierauf die zeitsteuernde Verbundmembran(V) aufgebracht. Der Kern kann einen oder mehrere Wirkstoffe in Kombination mit einem oder mehreren Absorptionsförderern, exemplarisch seien genannt 5-Methoxysalicylsäure, Salicylsäure und/oder Netzmitteln, z. B. Polysorbate, Blockcopolymere aus Ethylenoxid und Propylenoxid, ethoxylierte Glyceride und/oder Solubilisatoren enthalten. Auf die Verbundmembran (V) kann wiederum eine weitere Wirkstoffschicht, gegebenfalls auch eine zusätzliche Säureschicht und eine weitere Verbundmembran aufgebracht werden.
Die Menge und die Natur der aufgetragenen Säure in Zusammenhang mit der Durchlässigkeit der Verbundschicht ist bestimmend für die Dauer der Lagphase, das heißt der Phase ohne Wirkstofffrei-setzung. Die Durchlässigkeit der Verbundmembran kann in weiten Grenzen in Abhängigkeit von ihrer Zusammensetzung, z.B. durch Einsatz pH-sensitiver Polymere, die bei unterschiedlichen pH-Werten löslich sind, Mischungen dieser Polymere untereinander und/oder mit pH-unabhängig auflösenden oder unlöslichen Polymeren, Einbau porenbildender Substanzen, im weiteren durch unterschiedliche Gesamtschichtdicke, unterschiedliche Schichtdickenverhältnisse der pH-sensitiven und der unlöslichen Komponente der Verbundmembran den jeweiligen Anfordernissen an die Lagphase und Natur der Wirkstoffe angepaßt werden.
Prinzipiell richtet sich die Lagphase bei vorgegebener Zusammensetzung und Dicke der Verbundschicht nach der Säuremenge, die die Auflösung des pH-sensitiven Anteils der Verbundschicht verhindert. Nachdem die Säureschicht erschöpft ist, bricht die Verbundmembran zusammen und der darunter liegende Wirkstoff wird entsprechend den Eigenschaften der direkt auf ihn aufgebrachten Membran frei. Fehlt diese Steuermembran, wird der Wirkstoff mit maximaler Geschwindigkeit freigesetzt.

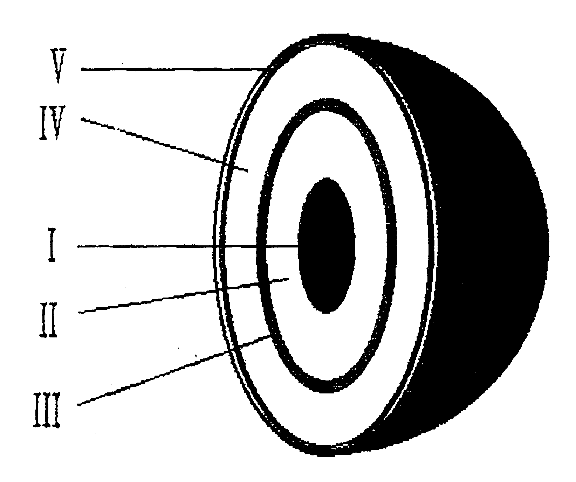

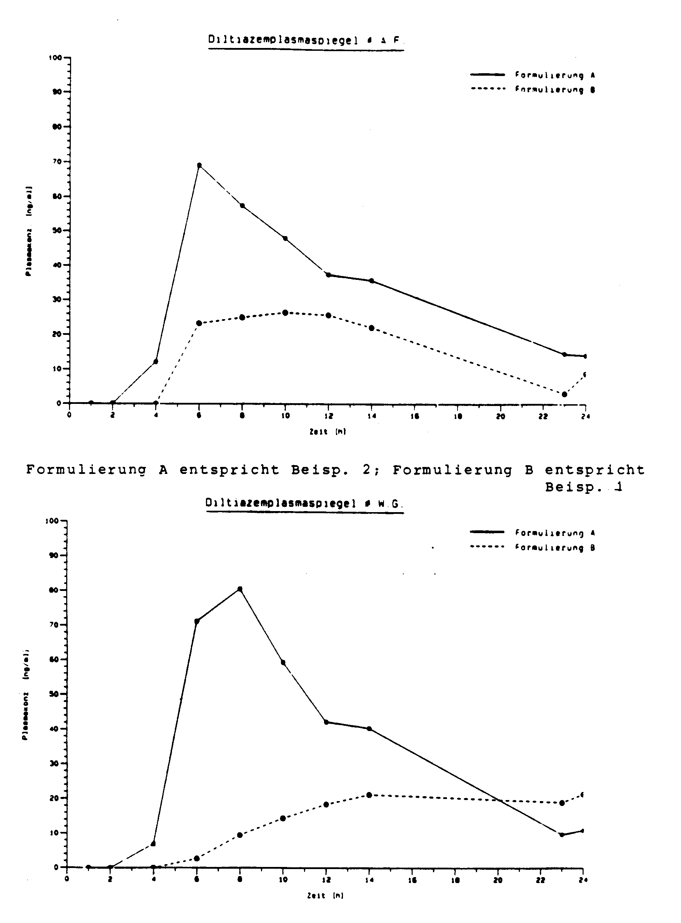

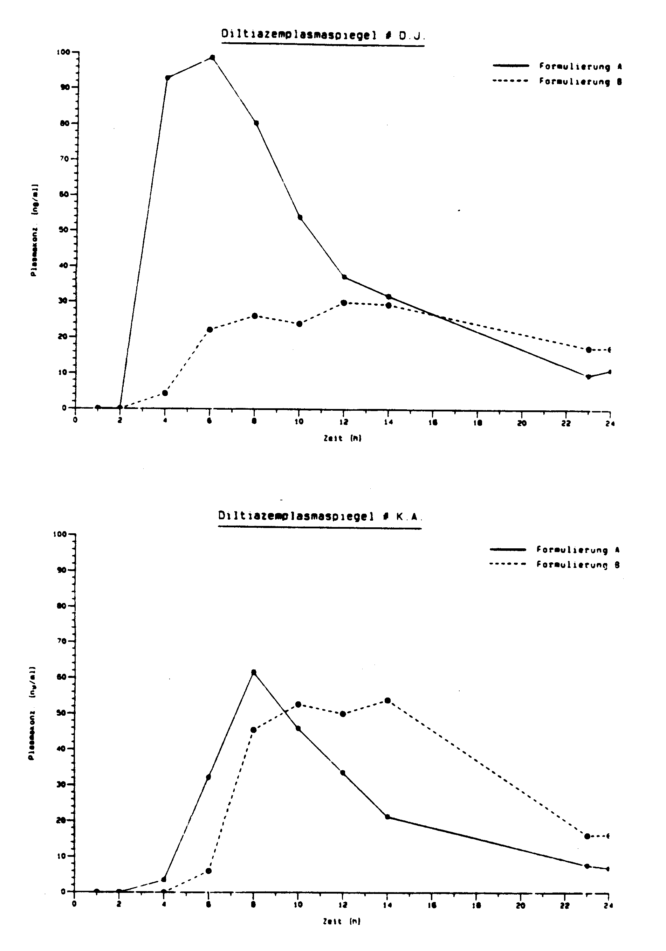

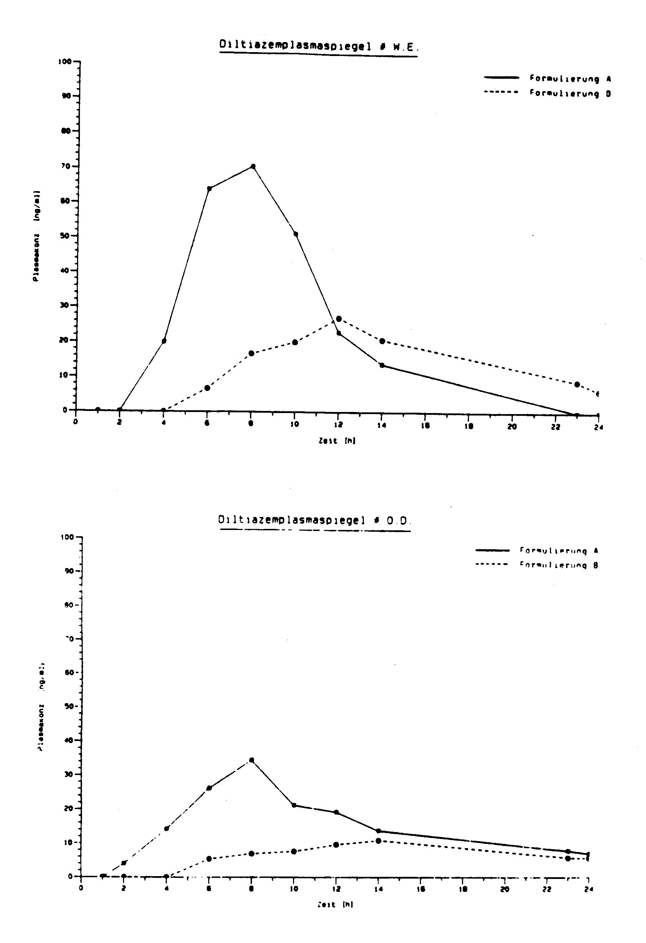

In Beispiel 1 ist eine Ausführungsform beschrieben, die etwa 4 Stunden nach Verlassen des Magens ihren Wirkstoff spontan freisetzt. Die in vivo-Prüfung an 6 Probanden zeigt deutlich, daß die gesamte Dosis reproduzierbar im Colon zur Resorption kam (Abb. 2). Im cross-over Versuch wurden Diltiazem-Pellets des Beispiels 2 appliziert. Die Pellets unterschieden sich von denen des Beispiels 1 durch das Fehlen der inneren freisetzungssteuernden Membran (III, bez. auf Abb. 1) und eine reduzierte Säuremenge (IV). Der Vergleich zeigt deutlich, daß die verringerte Säuremenge die Lagphase verkürzt. Der schnelle Anstieg der Plasmaspiegel ist eine Folge der fehlenden Membran (III). Da für die hier verwendeten Pellets eine Magenpassagezeit von etwa 1-2 Stunden anzunehmen ist (S.S. Davis, Top.Pharm.Sci., Proc. Int. Congr. Pharm. Sci. FIP, 43rd 1983 p. 205-215 D.D. Breimer and P. Speiser (Ed), Elsevier, Amsterdam; S.S. Davis et al. Int. J. Pharmaceutics, **21,** 331-340 (1984)), ergibt sich insgesamt in vivo eine Lagphase von etwa 5-6 Stunden für die Formulierung des Beispiels 1 und etwa 3-4 Stunden für die Formulierung des Beispiel 2.

Diese erste Formulierung ist geeignet
- in Kombination mit einer Teildosis, die über 6 Std nach der Einnahme freigesetzt wird, die einmal tägliche Gabe von Wirkstoffen wie z.B. Nifedipin, Diltiazem, Verapamil etc zu ermöglichen;
- Arzneistoffe, die im Magen oder Dünndarm labil sind wie etwa Peptidhormone, z.B. GH-RH, LH-RH, Insulin, Calcitonin, Wachstumshormone u.a. gezielt und reproduzierbar ins Colon zu transportieren und hier freizusetzen. Gerade Peptidpharmaka können zur Erhöhung der systemischen Verfügbarkeit in Kombination mit Resorptionsförderern wie 5-Methoxysalicylsäure, Salicylsäure u.a. kombiniert werden;
- Arzneistoffe, die in den frühen Morgenstunden vor dem Aufstehen in wirksamer Plasmakonzentration vorhanden sein sollen, wie z.B. Antiasthmatica, Antirheumatica, Antiphlogistica und Nitrate abends zuvor zu applizieren. Die Wirkstofffreisetzung ruht während der Nachtstunden, in denen keine therapeutische Plasmakonzentration erforderlich ist, um dann in den frühen Morgenstunden anzusteigen und die Patienten vor z.B. Asthmaanfällen oder Angina Pectoris Attacken während oder kurz vor der Aufwachphase zu schützen;
- Arzneistoffe, die im Colonbereich lokal wirken sollen, z.B. Antibiotika, Antiphlogistica, Spasmolytica u.a., gezielt an das Zielorgan zu bringen.

Die Formulierung in Beispiel 2 ist geeignet, in Kombination mit einer sofort freisetzenden Teildosis fluktuierende Plasmaspiegel zu erzeugen. Die in vivo Ergebnisse zeigen, daß die Freisetzung des Wirkstoffes im Dünndarm erfolgt und die Resorption nicht verlangsamt ist. Die Kombination entspräche der zweimaligen Applikation einer schnellfreisetzenden Form im Abstand von 4 Stunden. Dies ist eine Forderung, die z.B. an eine Therapie mit organischen Nitraten gestellt wird. Dadurch kann die Entstehung von Toleranz vermieden werden.

Das Beispiel 3 mit dem Wirkstoff Isosorbiddinitrat macht deutlich, daß die Lagphase innerhalb der physiologisch zu erwartenden pH-Werte unabhängig vom umgebenden Milieu ist (Vergleich Wirkstofffreisetzung 1 und 2). Die Milieubedingungen sind so gewählt, daß die Auflösung des pH-sensitiven Anteiles der Verbundmembran ohne die erfindungsgemäße Zusammensetzung eintreten müßte.
Beispiel 4 zeigt den Einfluß der Zusammensetzung auf die in vitro-Freisetzung anhand von Isosorbiddinitratpellets, bei denen im Vergleich zu Beispiel 3 die Säuremenge halbiert, der pH-sensitive Anteil der Verbundmembran jedoch verdoppelt ist. Die resultierende Lagphase ist gegenüber Beispiel 3 halbiert. Dieses zeigt, daß innerhalb der Verbundmembran nicht die Dicke der pH-sensitiven Schicht, sondern die eingesetzte Säuremenge zusammen mit der Verbundmembran für die Zeitsteuerung notwendig ist. Zum Vergleich ist in Beispiel 5 die Freisetzung von Pellets lediglich mit dem pH-sensitiven Anteil der Verbundmembran dargestellt. Eine Lagphase und damit eine zeitgesteuerte Wirkstofffreisetzung ist nicht zu erkennen.

### Beispiele

### Beispiel 1

| *Zusammensetzung* | |
|---|---|
| 1 Diltiazem-HCl | 50,0 g |
| 2 Zuckerkügelchen | 50,0 g |
| 3 Hydroxypropylcellulose | 1,0 g |
| 4 Celluloseacetatphthalat | 7,5 g |
| 5 Bernsteinsäure | 25,0 g |
| 6 Ethylcellulose | 6,0 g |
| 7 Hydroxypropylcellulose | 6,0 g |
| 8 Celluloseacetatphthalat | 7,5 g |
| 9 Ethylcellulose | 1,0 g |

### Herstellung

Auf die Zuckerkügelchen (2) wird der Wirkstoff (1) in einem geeigneten Kessel mittels einer Klebstofflösung, bestehend aus Klebstoff (3), gelöst in einem geeigneten Lösemittel, aufgebracht. Auf die Wirkstoffschicht wird eine Membran aus Celluloseacetatphthalat (4) und hierauf eine Schicht Säure (5) in der Wirbelschicht mit Hilfe einer Klebstofflösung/-dispersion aus 6 und 7 in einem geeigneten Löse- bzw. Dispersionsmittel aufgebracht. Auf die Säureschicht wiederum wird ebenfalls in der Wirbelschicht die Verbundmembran aus Celluloseacetatphthalat (8) und Ethylcellulose (9) in einem geeigneten Löse- bzw. Dispersionsmittel aufgebracht.

### Wirkstofffreisetzung

Methode: Rotating Bottle NF XIII, modifiziert
pH Wechsel nach 4 Stunden von pH=5,5 nach pH=7,5

| Zeit(h) | freigesetzter Wirkstoff | |
|---|---|---|
| | pH 5,5 | pH 7,5 |
| 2 | 0,2 % | |
| 4 | 0,7 % | |
| 6 | | 7,0 % |
| 8 | | 22,0 % |
| 10 | | 39,0 % |

### Beispiel 2

Die Herstellung der Diltiazem-Pellets erfolgt wie in Beispiel 1. Die freisetzungssteuernde Membran aus Celluloseacetatphthalat direkt über der Wirkstoffschicht entfällt jedoch. Die Bernsteinsäuremenge ist auf 20% reduziert.

| *Zusammensetzung* | |
|---|---|
| 1 Diltiazem-HCl | 50,0 g |
| 2 Zuckerkügelchen | 50,0 g |
| 3 Hydroxypropylcellulose | 1,0 g |
| 4 Bernsteinsäure | 5,0 g |
| 5 Ethylcellulose | 6,0 g |
| 6 Hydroxypropylcellulose | 6,0 g |
| 7 Celluloseacetatphthalat | 7,5 g |
| 8 Ethylcellulose | 1,0 g |

### Wirkstofffreisetzung

Methode: Rotating Bottle NF XIII
pH- Wechsel nach 4 Stunden von pH=5,5 nach pH=7,5

| Zeit (h) | freigesetzter Wirkstoff | |
|---|---|---|
| | pH 5,5 | pH 7,5 |
| 2 | 0,4 % | |
| 4 | 14,7 % | |
| 6 | | 63,0 % |
| 8 | | 102,0 % |

### Beispiel 3

| *Zusammensetzung* | |
|---|---|
| 1 Isosorbiddinitrat/Lactose 40/60 | 83,3 g |
| 2 Zuckerkügelchen | 50,0 g |
| 3 Hydroxypropylcellulose | 3,3 g |
| 4 Bernsteinsäure | 70,0 g |
| 5 Ethylcellulose | 8,4 g |
| 6 Hydroxypropylcellulose | 8,4 g |
| 7 Eudragit® L | 15,4 g |
| 8 Triacetin (Glyceryltriacetat) | 2,4 g |
| 9 Talkum | 2,7 g |
| 10 Ethylcellulose | 2,5 g |
| 11 Macrogol 1500 (PEG 1500) | 0,25g |
| 12 Talkum | 0,25g |

### Herstellung

Auf die Zuckerkügelchen (2) wird eine Mischung aus Isosorbiddinitrat und Lactose (1) in einem Kessel mittels einer Klebstofflösung, bestehend aus dem Klebstoff (3) und einem geeigneten Lösemittel, aufgebracht. Hierauf wird in der Wirbelschicht die Säure (4) mittels einer Klebstofflösung aus den Klebstoffen (5) und (6) aufgetragen. Auf die Säureschicht wird, ebenfalls in der Wirbelschicht, die Verbundmembran aus den Stoffen (7) bis (12) aufgebracht.

### Wirkstofffreisetzung 1

Methode: Rotating Bottle NF XIII, mod.
pH-Wechsel nach 4 Stunden von pH=5,5 nach pH=6,0

| Zeit (h) | freigesetzter Wirkstoff | |
|---|---|---|
| | pH 5,5 | pH 6,0 |
| 2 | 3,5 % | |
| 4 | 8,2 % | |
| 6 | | 39,2 % |

### Wirkstofffreisetzung 2

Methode: Rotating Bottle NF XIII, mod

| Zeit (h) | freigesetzter Wirkstoff pH-Wert = 6,0 |
|---|---|
| 1 | 1,3 % |
| 2 | 2,9 % |
| 3 | 4,5 % |
| 4 | 9,3 % |
| 5 | 24,8 % |
| 6 | 44,2 % |

### Beispiel 4

| *Zusammensetzung* | |
|---|---|
| 1 Isosorbiddinitrat/Lactose 40/60 | 83,3 g |
| 2 Zuckerkügelchen | 50,0 g |
| 3 Hydroxypropylcellulose | 3,3 g |
| 4 Bernsteinsäure | 35,0 g |
| 5 Ethylcellulose | 8,4 g |
| 6 Hydroxypropylcellulose | 8,4 g |
| 7 Eudragit® L | 10,9 g |
| 8 Triacetin (Glyceryltriacetat) | 2,25g |
| 9 Talkum | 2,25g |
| 10 Ethylcellulose | 2,5 g |
| 11 Macrogol 1500 (PEG 1500) | 0,25g |
| 12 Talkum | 0,5 g |

Die Herstellung erfolgt analog. Die Bernsteinsäuremenge ist halbiert, die Eudragit® L Menge verdoppelt.

### Wirkstofffreisetzung

Methode: Rotating Bottle NF XIII
pH-Wechsel nach 4 Stunden von pH=6,0 nach pH=7,5

| Zeit (h) | freigesetzter Wirkstoff | |
|---|---|---|
| | pH 6,0 | pH 7,5 |
| 2 | 17,9 % | |
| 4 | 70,9 % | |
| 6 | | 95,3 % |

### Beispiel 5

Die Herstellung der Isosorbiddinitratpellets erfolgt wie in Beispiel 4. Der Ethylcelluloseanteil der Verbundmembran entfällt jedoch.

### Wirkstofffreisetzung 1

Methode: Rotating Bottle NF XIII
pH-Wechsel nach 4 Stunden von pH=6,0 nach pH=7,5

| Zeit (h) | freigesetzter Wirkstoff | |
|---|---|---|
| | pH 6,0 | pH 7,5 |
| 2 | 54,2 % | |
| 4 | 92,8 % | |
| 6 | | 102,9 % |

### Wirkstofffreisetzung 2

Methode: Rotating Bottle NF XIII, mod.
pH-Wechsel nach 4 Stunden von pH=5,5 nach pH=7,5

| Zeit (h) | pH-Wert | |
|---|---|---|
| | 5,5 | 7,5 |
| 2 | 2,0 % | |
| 4 | 4,5 % | |
| 6 | | 68,9 % |

## Patentansprüche (Patentansprüche für folgende(n) Vertragsstaat(en): AT, BE, CH, DE, FR, GB, IT, LI, LU, NL, SE)

1. Oral zu verabreichende pharmazeutische Zubereitung, bestehend aus einem Kern und ihn umhüllende Schichten, dadurch gekennzeichnet, daß einen Kern (I) eine wirkstoffhaltige Schicht (II), enthaltend mindestens eine pharmakologisch aktive Substanz, eine pH-unabhängig oder pH-abhängig lösliche Membran (III), eine aus mindestens einer physiologisch unbedenklichen Säure bestehende Säureschicht (IV), und eine aus zwei diskreten Schichten, deren eine von einem pH-sensitiven Polymer und deren andere von einem unlöslichen Polymer gebildet wird, bestehende Verbundmembran (V) umhüllen.

2. Oral zu verabreichende pharmazeutische Zubereitung nach Anspruch 1, dadurch gekennzeichnet, daß der Kern selbst aus mindestens einer pharmakologisch aktiven Substanz besteht.

3. Oral zu verabreichende pharmazeutische Zubereitung nach Ansprüchen 1 und 2, dadurch gekennzeichnet, daß die Säureschicht aus Natriumdihydrogenphosphat, Citronensäure, Weinsäure, Bernsteinsäure oder Fumarsäure, ohne darauf beschränkt zu sein, und/oder Mischungen davon besteht.

4. Oral zu verabreichende pharmazeutische Zubereitung nach Ansprüchen 1 - 3, dadurch gekennzeichnet, daß die Membran Celluloseacetatphtalat ist.

5. Oral zu verabreichende pharmazeutische Zubereitung nach Ansprüchen 1 - 4, dadurch gekennzeichnet, daß das pH-sensitive Polymer der Verbundmembran aus Copolymeren aus Methacrylsäure und Methacrylsäuremethylester, Carboxymethylethylcellulose, Celluloseacetatphtalat, Celluloseacethylphtalat, Cellulosediacethylphtalat, Cellulosetriacethylphtalat, Hydroxypropylmethylcellulosephtalat, Natriumcelluloseacetatphtalat, Celluloseesterphtalat, Celluloseetherphtalat, Celluloseesteretherphtalat, Hydroxypropylcellulosephtalat, Methylcellulosephtalat, Keratin, Keratin-Sandarac-Tolubalsam, Salol, Salol-ß-naphtylbenzoat und Acetotannin oder Salol mit Perubalsam, ohne darauf beschränkt zu sein, und/oder Mischungen dieser Polymere untereinander und/oder löslichen oder unlöslichen Polymeren besteht.

6. Oral zu verabreichende pharmazeutische Zubereitung nach Ansprüchen 1 - 5, dadurch gekennzeichnet, daß das unlösliche Polymer der Verbundmembran Celluloseether, Copolymerisat aus Acryl- und Methacrylsäureestern mit quartären Ammoniumgruppen oder Polyvinylacetat, ohne darauf beschränkt zu sein, und/oder Mischungen davon und/oder Anteile von löslichen Polymeren enthaltend, ist.

7. Oral zu verabreichende pharmazeutische Zubereitung nach Ansprüchen 1 - 6, dadurch gekennzeichnet, daß die Verbundmembran porenbildende Substanzen enthält.

8. Oral zu verabreichende pharmazeutische Zubereitung nach Ansprüchen 1 - 7, dadurch gekennzeichnet, daß der unlösliche Polymeranteil der Verbundmembran 1 - 30 Gew.-% beträgt.

9. Oral zu verabreichende pharmazeutische Zubereitung nach Ansprüchen 1 - 8, dadurch gekennzeichnet, daß die wirkstoffhaltige Schicht zusätzlich zu mindestens einer pharmakologisch aktiven Substanz mindestens einen Absorptionsförderer und/oder mindestens einen Solubilisator enthält.

10. Oral zu verabreichende pharmazeutische Zubereitung, bestehend aus einem Kern und ihn umhüllende Schichten, dadurch gekennzeichnet, daß einen Kern (I) eine wirkstoffhaltige Schicht (II), enthaltend mindestens eine pharmakologisch aktive Substanz, eine aus mindestens einer physiologisch unbedenklichen Säure bestehende Säureschicht (IV) und eine aus zwei diskreten Schichten, deren eine von einem pH-sensitiven Polymer und deren andere von einem unlöslichen Polymer gebildet wird, bestehende Verbundmembran (V) umhüllen.

11. Oral zu verabreichende pharmazeutische Zubereitung nach Ansprüchen 1 - 10, dadurch gekennzeichnet, daß die Arzneiform eine Tablette, ein Pellet, eine Kapsel, ein Granulum oder ein Dragee ist.

12. Verfahren zur Herstellung einer oral zu verabreichenden pharmazeutischen Zubereitung nach Ansprüchen 1 - 11, dadurch gekennzeichnet, daß auf einen physiologisch unbedenklichen Kern (I) mindestens eine pharmakologisch aktive Substanz (II) aufgebracht wird, auf die gegebenenfalls eine Membranschicht (III) und hierauf eine Säureschicht (IV) aufgetragen wird, auf die die Verbundmembran (V), bestehend aus zwei diskreten Schichten, deren eine von einem pH-sensitiven Polymer und deren andere von einem unlöslichen Polymer gebildet wird, aufgebracht wird.

13. Verfahren zur Herstellung einer oral zu verabreichenden pharmazeutischen Zubereitung nach Anspruch 12, dadurch gekennzeichnet, daß die verschiedenen Schichten durch Agglomerieren, Aufsprüchen in der Wirbelschicht, Auftragen per Hand, durch Pulverdosierer, durch Aufpressen, Verpressen, Aufkleben, Extrudieren, Tauchen und/oder Aufwalzen auf den wirkstoffhaltigen Kern aufgebracht werden.

14. Oral zu verabreichende pharmazeutische Zubereitung nach Ansprüchen 1 - 13 zur Therapie von Krankheiten.

## Patentansprüche (Patentansprüche für folgende(n) Vertragsstaat(en): ES, GR)

1. Verfahren zur Herstellung einer oral zu verabreichenden pharmazeutischen Zubereitung, bestehend aus einem Kern und ihn umhüllende Schichten, dadurch gekennzeichnet, daß auf einen Kern (I) eine wirkstoffhaltige Schicht (II), enthaltend mindestens eine pharmakologisch aktive Substanz, aufgebracht wird, auf die eine pH-unabhängig oder pH-abhängig lösliche Membran (III) und hierauf eine aus mindestens einer physiologisch unbedenklichen Säure bestehende Säureschicht (IV) aufgetragen wird, auf die eine Verbundmembran (V), bestehend aus zwei diskreten Schichten, deren eine von einem pH-sensitiven Polymer und deren andere von einem unlöslichen Polymer gebildet wird, aufgebracht wird.

2. Verfahren zur Herstellung einer oral zu verabreichenden pharmazeutischen Zubereitung nach Anspruch 1, dadurch gekennzeichnet, daß der Kern selbst aus mindestens einer pharmakologisch aktiven Substanz gebildet wird.

3. Verfahren zur Herstellung einer oral zu verabreichenden pharmazeutischen Zubereitung nach Ansprüchen 1 und 2, dadurch gekennzeichnet, daß die Säureschicht aus Natriumdihydrogenphosphat, Citronensäure, Weinsäure, Bernsteinsäure oder Fumarsäure, ohne darauf beschränkt zu sein, und/oder Mischungen davon, gebildet wird.

4. Verfahren zur Herstellung einer oral zu verabreichenden pharmazeutischen Zubereitung nach Ansprüchen 1 - 3, dadurch gekennzeichnet, daß die Membran aus Celluloseacetatphtalat gebildet wird.

5. Verfahren zur Herstellung einer oral zu verabreichenden pharmazeutischen Zubereitung nach Ansprüchen 1 - 4, dadurch gekennzeichnet, daß das pH-sensitive Polymer der Verbundmembran aus Copolymeren aus Methacrylsäure und Methacrylsäuremethylester, Carboxymethylethylcellulose, Celluloseacetatphtalat, Celluloseacethylphtalat, Cellulosediacethylphtalat, Cellulosetriacethylphtalat, Hydroxypropylmethylcellulosephtalat, Natriumcelluloseacetatphtalat, Celluloseesterphtalat, Celluloseetherphtalat, Celluloseesteretherphtalat, Hydroxypropylcellulosephtalat, Methylcellulosephtalat, Keratin, Keratin-Sandarac-Tolubalsam, Salol, Salol-ß-naphtylbenzoat und Acetotannin oder Salol mit Perubalsam, ohne darauf beschränkt zu sein, und/oder Mischungen dieser Polymere untereinander und/oder löslichen oder unlöslichen Polymeren gebildet wird.

6. Verfahren zur Herstellung einer oral zu verabreichenden pharmazeutischen Zubereitung nach Ansprüchen 1 - 5, dadurch gekennzeichnet, daß das unlösliche Polymer der Verbundmembran aus Celluloseether, Copolymerisat aus Acryl- und Methacrylsäureestern mit quartären Ammoniumgruppen oder Polyvinylacetat, ohne darauf beschränkt zu sein, und/oder Mischungen davon und/oder Anteile von löslichen Polymeren enthaltend, gebildet wird.

7. Verfahren zur Herstellung einer oral zu verabreichenden pharmazeutischen Zubereitung nach Ansprüchen 1 - 6, dadurch gekennzeichnet, daß die Verbundmembran unter Zusetzen porenbildender Substanzen gebildet wird.

8. Verfahren zur Herstellung einer oral zu verabreichenden pharmazeutischen Zubereitung nach Ansprüchen 1 - 7, dadurch gekennzeichnet, daß die Verbundmembran mit einem unlöslichen Polymeranteil von 1 - 30 Gew.-% gebildet wird.

9. Verfahren zur Herstellung einer oral zu verabreichenden pharmazeutischen Zubereitung nach Ansprüchen 1 - 8, dadurch gekennzeichnet, daß der wirkstoffhaltigen Schicht zusätzlich zu mindestens einer pharmakologisch aktiven Substanz mindestens ein Absorptionsförderer und/oder mindestens ein Solubilisator zugegeben werden.

10. Verfahren zur Herstellung einer oral zu verabreichenden pharmazeutischen Zubereitung, bestehend aus einem Kern und ihn umhüllende Schichten, dadurch gekennzeichnet, daß auf einen Kern (I) eine wirkstoffhaltige Schicht (II), enthaltend mindestens eine pharmakologisch aktive Substanz, aufgebracht wird, auf die eine aus mindestens einer physiologisch unbedenklichen Säure bestehende Säureschicht (IV) aufgetragen wird, auf die eine Verbundmembran (V), bestehend aus zwei diskreten Schichten, deren eine von einem pH-sensitiven Polymer und deren andere von einem unlöslichen Polymer gebildet wird, aufgebracht wird.

11. Verfahren zur Herstellung einer oral zu verabreichenden pharmazeutischen Zubereitung nach Ansprüchen 1 - 10, dadurch gekennzeichnet, daß die verschiedenen Schichten durch Agglomerieren, Aufsprühen in der Wirbelschicht, Auftragen per Hand, durch Pulverdosierer, durch Aufpressen, Verpressen, Aufkleben, Extrudieren, Tauchen und/oder Aufwalzen auf den wirkstoffhaltigen Kern aufgebracht werden.

12. Verfahren zur Herstellung einer oral zu verabreichenden pharmazeutischen Zubereitung nach Ansprüchen 1 - 11, dadurch gekennzeichnet, daß die verfahrensgemäß erhaltenen umhüllten Kerne zu Tabletten oder Dragees verpreßt werden oder in Kapseln abgefüllt werden.

13. Verfahren zur Herstellung einer oral zu verabreichenden pharmazeutischen Zubereitung nach Ansprüchen 1 - 12 zur Verwendung zur Therapie von Krankheiten.

## Claims (Claims for the following Contracting State(s): AT, BE, CH, DE, FR, GB, IT, LI, LU, NL, SE)

1. Pharmaceutical preparation bo be administered orally, comprising a nucleus and layers surrounding it, characterized in that a nucleus (I) is enveloped by an active substance layer (II) containing at least one pharmacologically active substance, a pH independent or pH-dependent soluble membrane layer (III), an acid layer comprising at least one physiologically safe acid (IV) and a compound membrane layer (V) consisting of two discrete layers of which one is consisting of a pH-sensitive polymer and the other layer is consisting of an insoluble polymer.

2. Pharmaceutical preparation to be administered orally according to claim 1, characterized in that the nucleus consists of at least one pharmacologically active substance.

3. Pharmaceutical preparation to be administered orally according to claims 1 and 2, characterized in that the acid layer comprises sodium dihydrogenphosphate, citric acid, tartaric acid, succinic acid, fumaric acid without being restricted to this, and/or mixtures therefrom.

4. Pharmaceutical preparation to be administered orally according to claims 1 to 3, characterized in that the membrane is a cellulose acetate phthalate.

5. Pharmaceutical preparation to be administered orally according to claims 1 to 4, characterized in that the pH sensitive polymer of the compound membrane comprises copolymers of methacrylic acid and methacrylic acid methylester, carboxymethylethyl cellulose, cellulose acetate phthalate, cellulose acetylphthalate, cellulose diacetylphthalate, cellulose triacetylphthalate, hydroxypropylmethyl cellulose phthalate, sodium cellulose acetatephthalate, cellulose esterphthalate, cellulose etherphthalate, cellulose esteretherphthalate, hydroxypropyl cellulose phthalate, methyl cellulose phthalate, keratin, keratin-sandarac-tolubalsam, salol, salol-ß-naphtylbenzoate and acetotannin or salol with perubalsam, without being restricted to this and/or mixtures of these polymers among one another and/or soluble or insoluble polymers.

6. Pharmaceutical preparation to be administered orally according to claims 1 to 5, characterized in that the insoluble polymer of the compound membrane is cellulose ether, a copolymerisate of acryl- and methacryl acid esters with quarternary ammonium groups or polyvinylacetate, without being restricted to this, and/or mixtures thereof and/or containing portions of soluble polymers.

7. Pharmaceutical preparation to be administered orally according to claims 1 to 6, characterized in that the compound membrane contains pore-forming substances.

8. Pharmaceutical preparation to be administered orally according to claims 1 to 7, characterized in that the polymer portion of the insoluble layer of the compound membrane amounts to 1 to 30 weight percent.

9. Pharmaceutical preparation to be administered orally according to claims 1 to 8, characterized in that the layer containing the active substance, in addition to at least one pharmacologically active substance, contains at least one absorption enhancer and/or at least a solubilizer.

10. Pharmaceutical preparation to be administered orally, comprising a nucleus and layers surrounding it, characterized in that, a nucleus (I) is enveloped by an active substance layer (II) containing at least one pharmacologically active substance, an acid layer comprising at least one physiologically safe acid (IV) and a compound membrane (V) consisting of two discrete layers of which one is consisting of a pH-sensitive polymer and the other layer is consisting of an insoluble polymer.

11. Pharmaceutical preparation to be administered orally according to claims 1 to 10, characterized in that the medicine type is a tablet, a pellet, a capsule, a granule, or a dragee.

12. Method for the manufacture of a pharmaceutical preparation to be administered orally according to claims 1 to 11, characterized in that, on a physiologically safe nucleus (I), at least one pharmacologically active substance (II) is applied, on which, if needed, a membrane layer (III) and on the latter an acid layer (IV) is applied on which the compound membrane (V) consisting of two discrete layers of which one is consisting of a pH-sensitive polymer and the other layer is consisting of a insoluble polymer is applied.

13. Method for the manufacture of a pharmaceutical preparation to be administered orally according to claim 12 characterized in that the various layers are applied by means of agglomeration, spraying-on in a fluidized bed vessel, manual application, by means of a powder dosing device, by pressing, punching, gluing, extrusion, immersion, and/or rolling-on onto the nucleus containing the active substance.

14. Pharmaceutical preparation to be administered orally according to claims 1 to 13 for the therapy of illnesses.

## Claims (Claims for the following Contracting State(s): ES, GR)

1. Process for the manufacture of a pharmaceutical preparation to be administered orally, comprising a nucleus and layers surrounding it, characterized in that, on a nucleus (I) an active substance layer (II), containing at least one pharmacologically active substance is applied on which a pH-independent or a pH-dependent soluble membrane (III) is applied, on which an acid layer comprising at least one physiologically safe acid (IV) is applied on which the compound membrane (V) consisting of two discrete layers of which one is consisting of a pH-sensitive polymer and the other layer is consisting of an insoluble polymer, is applied.

2. Process for the manufacture of a pharmaceutical preparation to be administered orally according to claim 1, characterized in that the nucleus is formed by at least one pharmacologically active substance.

3. Process for the manufacture of a pharmaceutical preparation to be administered orally according to claims 1 and 2, characterized in that the acid layer is formed by sodium dihydrogenphosphate, citric acid, tartaric acid, succinic acid, fumaric acid without being restricted to this, and/or mixtures therefrom.

4. Process for the manufacture of a pharmaceutical preparation to be administered orally according to claims 1 to 3, characterized in that the membrane is formed by a cellulose acetate phthalate

5. Process for the manufacture of a pharmaceutical preparation to be administered orally according to claims 1 to 4, characterized in that the pH sensitive polymer of the compound membrane is formed by copolymers of methacrylic acid and methacrylic acid methylester, carboxymethylethyl cellulose, cellulose acetate phthalate, cellulose acetylphthalate, cellulose diacetylphthalate, cellulose triacetylphthalate, hydroxypropylmethyl cellulose phthalate, sodium cellulose acetatephthalate, cellulose esterphthalate, cellulose etherphthalate, cellulose esteretherphthalate, hydroxypropyl cellulose phthalate, methyl cellulose phthalate, keratin, keratin-sandarac-tolubalsam, salol, salol-ß-naphtylbenzoate and acetotannin or salol with perubalsam, without being restricted to this and/or mixtures of these polymers among one another and/or soluble or insoluble polymers.

6. Process for the manufacture of a pharmaceutical preparation to be administered orally according to claims 1 to 5, characterized in that the insoluble polymer of the compound membrane is formed by cellulose ether, a copolymerisate of acryl- and methacryl acid esters with quarternary ammonium groups or polyvinylacetate, without being restricted to this, and/or mixtures thereof and/or containing portions of soluble polymers.

7. Process for the manufacture of a pharmaceutical preparation to be administered orally according to claims 1 to 6, characterized in that the compound membrane is formed by adding pore-forming substances.

8. Process for the manufacture of a pharmaceutical preparation to be administered orally according to claims 1 to 7, characterized in that the polymer portion of the insoluble layer of the compound membrane is formed by amounts to 1 to 30 weight percent.

9. Process for the manufacture of a pharmaceutical preparation to be administered orally according to claims 1 to 8, characterized in that to the layer containing the active substance, is added at least one pharmacologically active substance, containing at least one absorption enhancer and/or at least a solubilizer.

10. Process for the manufacture of a pharmaceutical preparation to be administered orally comprising a nucleus and layers surrounding it, characterized in that on a nucleus (I) an active substance layer (II), containing at least one pharmacologically active substance is applied on which an acid layer comprising at least one physiologically safe acid (IV) is applied, on which the compound membrane (V) consisting of two discrete layers of which one is consisting of a pH-sensitive polymer and the other layer is consisting of an insoluble polymer, is applied.

11. Process for the manufacture of a pharmaceutical preparation to be administered orally according to claims 1 to 10, characterized in that the various layers are applied by means of agglomeration, spraying-on in a fluidized bed vessel, manual application, by means of a powder dosing device, by pressing, punching, gluing, extrusion, immersion, and/or rolling-on onto the nucleus containing the active substance.

12. Process for the manufacture of a pharmaceutical preparation to be administered orally according to claims 1 to 11, characterized in that the medicine type is a tablet, a pellet, a capsule, a granule, or a dragee.

13. Process for the manufacture of a pharmaceutical preparation to be administered orally according to claims 1 to 12 for the therapy of illnesses.

## Revendications (Revendications pour l'(les) Etat(s) contractant(s) suivant(s): AT, BE, CH, DE, FR, GB, IT, LI, LU, NL, SE)

1. Préparation pharmaceutique pour administration orale, laquelle est constituée d'un noyau et de couches l'enrobant, et caractérisée en ce que le noyau (I) est enrobé d'une couche d'agent actif (II) constituée d'au-moins une substance pharmacologiquement active, d'une membrane (III) dont la solubilité est dépendante ou indépendante du pH, d'une couche acide (IV) constituée d'au-moins un acide physiologiquement neutre, et d'une membrane composite (V) constituée de deux couches discrètes, l'une étant formée par un polymère sensitif au pH et l'autre par un polymère insoluble.

2. Préparation pharmaceutique pour administration orale, selon la revendication 1, caractérisée en ce que le noyau lui-même est constitué d'au-moins une substance pharmacologiquement active.

3. Préparation pharmaceutique pour administration orale selon les revendications 1 et 2, caractérisée en ce que la couche acide est constituée de dihydrogénophosphate de sodium, d'acide citrique, d'acide tartrique, d'acide succinique ou d'acide fumarique, sans pour autant être limitée à ces constituants, et/ou de leurs mélanges.

4. Préparation pharmaceutique pour administration orale selon les revendications 1-3, caractérisée en ce que la membrane est constituée d'acétophtalate de cellulose.

5. Préparation pharmaceutique pour administration orale selon les revendications 1-4, caractérisée en ce que le polymère sensitif au pH de la membrane composite est constitué de copolymères d'acide méthacrylique et de méthylester d'acide méthacrylique, de cellulose de carboxyméthyléthyle, d'acétophtalate de cellulose, de phtalate d'acétyle de cellulose, de phtalate de diacétyle de cellulose, de phtalate de triacétyle de cellulose, de phtalate de cellulose d'hydroxypropylméthyle, d'acétophtalate de cellulose de sodium, de phtalate d'ester de cellulose, de phtalate d'éther de cellulose, de phtalate d'étherester de cellulose, de phtalate d'hydroxypropyle de cellulose, de phtalate de méthyle de cellulose, de kératine, de baume de Tolu, de kératine sandaraque, de salol, de benzoate de salol-ß-naphtyle, d'acétotanin, ou de salol au baume du Pérou, sans pour autant être limité à ces composants, et/ou composé de mélanges de ces polymères entre eux et/ou de polymères solubles ou insolubles.

6. Préparation pharmaceutique pour administration orale selon les revendications 1- 5, caractérisée en ce que le polymère insoluble de la membrane composite est un éther de cellulose, un copolymérisat d'esters d'acides acrylique et méthacrylique avec des groupes d'ammonium quaternaires ou d'acétate de polyvinyle, sans pour autant être limité à ces composants, et/ou comportant des mélanges de ceux-ci et/ou des éléments polymériques solubles.

7. Préparation pharmaceutique pour administration orale selon les revendications 1-6, caractérisée en ce que la membrane composite comporte des substances porogènes.

8. Préparation pharmaceutique pour administration orale, selon les revendications 1-7 caractérisée en ce que les éléments polymériques insolubles de la membrane composite constituent 1-30 % en poids.

9. Préparation pharmaceutique pour administration orale selon les revendications 1-8 caractérisée en ce que que la couche d'agent actif comporte, outre un minimum d'une substance pharmacologiquement active, au moins un adjuvant d'absorption, et/ou au moins un solubilisateur.

10. Préparation pharmaceutique pour administration orale constituée d'un noyau et de couches l'enrobant, caractérisée en ce que le noyau (I) est enrobé par une couche d'agent actif (II) constituée d'au-moins une substance pharmacologiquement active, par une couche acide (IV) constituée d'au-moins un acide physiologiquement neutre et par une membrane composite (V) constituée de deux couches discrètes dont l'une est formée par un polymère sensitif au pH et l'autre par un polymère insoluble.

11. Préparation pharmaceutique pour administration orale selon les revendication 1-10, caractérisée en ce que la forme galénique est une comprimé, un pellet, une capsule, un granulé ou une dragée.

12. Procédé de fabrication d'une préparation pharmaceutique pour administration orale selon les revendications 1-11, caractérisé en ce qu'un noyau (I) physiologiquement neutre est enrobé d'au-moins une substance pharmacologiquement active (II), elle-même éventuellement recouverte d'une couche membraneuse (III) puis d'une couche acide (IV) et enfin d'une membrane composite (V) comportant deux couches discrètes dont l'une est formée par un polymère sensitif au pH et l'autre par un polymère insoluble.

13. Procédé de fabrication d'une préparation pharmaceutique pour administration orale selon la revendication 12, caractérisé en ce que les différentes couches sont appliquées sur le noyau d'agent actif par agglomération, par pulvérisation, par application manuelle, par compression, par engluage, par extrusion, par immersion et/ou par compactage.

14. Préparation pharmaceutique pour administration orale selon les revendications 1-13, pour usage thérapeutique.

## Revendications (Revendications pour l'(les) Etat(s) contractant(s) suivant(s): ES, GR)

1. Procédé de fabrication d'une préparation pharmaceutique pour administration orale, laquelle est constituée d'un noyau et de couches l'enrobant, et caractérisé en ce que l'on enrobe le noyau (I) d'une couche d'agent actif (II) constituée d'au-moins une substance pharmacologiquement active, d'une membrane (III) dont la solubilité est dépendante ou indépendante du pH, d'une couche acide (IV) constituée d'au-moins un acide physiologiquement neutre, et d'une membrane composite (V) constituée de deux couches discrètes, l'une étant formée par un polymère sensitif au pH et l'autre par un polymère insoluble.

2. Procédé de fabrication d'une préparation pharmaceutique pour administration orale, selon la revendication 1, caractérisé en ce que le noyau lui-même est constitué à partir d'au-moins une substance pharmacologiquement active.

3. Procédé de fabrication d'une préparation pharmaceutique pour administration orale selon les revendications 1 et 2, caractérisé en ce que la couche acide est constituée à partir de dihydrogénophosphate de sodium, d'acide citrique, d'acide tartrique, d'acide succinique ou d'acide fumarique, sans pour autant être limitée à ces constituants, et/ou de leurs mélanges.

4. Procédé de fabrication d'une préparation pharmaceutique pour administration orale selon les revendications 1-3, caractérisé en ce que la membrane est constituée à partir d'acétophtalate de cellulose.

5. Procédé de fabrication d'une préparation pharmaceutique pour administration orale selon les revendications 1-4, caractérisé en ce que le polymère sensitif au pH de la membrane composite est constitué à partir de copolymères d'acide méthacrylique et de méthylester d'acide méthacrylique, de cellulose de carboxyméthyléthyle, d'acétophtalate de cellulose, de phtalate d'acétyle de cellulose, de phtalate de diacétyle de cellulose, de phtalate de triacétyle de cellulose, de phtalate de cellulose d'hydroxypropylméthyle, d'acétophtalate de cellulose de sodium, de phtalate d'ester de cellulose, de phtalate d'éther de cellulose, de phtalate d'étherester de cellulose, de phtalate d'hydroxypropyle de cellulose, de phtalate de méthyle de cellulose, de kératine, de baume de Tolu, de kératine sandaraque, de salol, de benzoate de salol-ß-naphtyle, d'acétotanin, ou de salol au baume du Pérou, sans pour autant être limité à ces composants, et/ou composé de mélanges de ces polymères entre eux et/ou de polymères solubles ou insolubles.

6. Procédé de fabrication d'une préparation pharmaceutique pour administration orale selon les revendications 1- 5, caractérisé en ce que le polymère insoluble de la membrane composite est formé à partir d'un éther de cellulose, d'un copolymérisat d'esters d'acides acrylique et méthacrylique avec des groupes d'ammonium quaternaires ou d'acétate de polyvinyle, sans pour autant être limité à ces composants, et/ou comportant des mélanges de ceux-ci et/ou des éléments polymériques solubles.

7. Procédé de fabrication d'une préparation pharmaceutique pour administration orale selon les revendications 1-6, caractérisé en ce que l'on réalise la membrane composite avec un apport de substances porogènes.

8. Procédé de fabrication d'une préparation pharmaceutique pour administration orale, selon les revendications 1-7 caractérisé en ce que les éléments polymériques insolubles de la membrane composite constituent 1-30 % en poids.

9. Procédé de fabrication d'une préparation pharmaceutique pour administration orale selon les revendications 1-8 caractérisé en ce qu'on ajoute à la couche d'agent actif, outre un minimum d'une substance pharmacologiquement active, au moins un adjuvant d'absorption, et/ou au moins un solubilisateur.

10. Procédé da fabrication d'une préparation pharmaceutique pour administration orale constituée d'un noyau et de couches l'enrobant, caractérisé en ce que l'on enrobe le noyau (I) d' une couche d'agent actif (II) constituée d'au-moins une substance pharmacologiquement active, d' une couche acide (IV) constituée d'au-moins un acide physiologiquement neutre et d'une membrane composite (V) constituée de deux couches discrètes dont l'une est formée par un polymère sensitif au pH et l'autre par un polymère insoluble.

11. Procédé de fabrication d'une préparation pharmaceutique pour administration orale selon les revendications 1-10, caractérisé en ce que les différentes couches sont appliquées sur le noyau d'agent actif par agglomération, par pulvérisation en lit fluidisé, par application manuelle, par doseur de poudre, par application avec pression, par compression, par engluage, par extrusion, par immersion et/ou par compactage.

12. Procédé de fabrication d'une préparation pharmaceutique pour administration orale selon les revendications 1-11, caractérisé en ce que les noyaux enrobés selon le procédé décrit subissent un compactage en comprimés ou en dragées ou sont emplis en capsules.

13. Procédé de fabrication d'une préparation pharmaceutique pour administration orale selon les revendications 1-12 pour usage thérapeutique.
